(19) Europäisches Patentamt
European Patent Office
Office européen des brevets

(11) Veröffentlichungsnummer: **0 087 712**
**B1**

(12) # EUROPÄISCHE PATENTSCHRIFT

(45) Veröffentlichungstag der Patentschrift: **07.05.86**

(51) Int. Cl.[4]: **C 07 C121/34**, C 07 C121/16, C 07 C 53/126

(21) Anmeldenummer: **83101639.9**

(22) Anmeldetag: **21.02.83**

(54) **Verfahren zur Herstellung von 3-Cyan-2-alkylalkanalen und die Verwendung von 3-Cyan-2-ethylhexanal zur Herstellung von 2-Propylpentansäure.**

(30) Priorität: **03.03.82 DE 3207614**
**03.03.82 DE 3207615**

(43) Veröffentlichungstag der Anmeldung: **07.09.83 Patentblatt 83/36**

(45) Bekanntmachung des Hinweises auf die Patenterteilung: **07.05.86 Patentblatt 86/19**

(84) Benannte Vertragsstaaten: **BE CH DE FR GB IT LI**

(56) Entgegenhaltungen: **US-A- 2 565 537**

(73) Patentinhaber: **BASF Aktiengesellschaft**
**Carl-Bosch-Strasse 38**
**D-6700 Ludwigshafen (DE)**

(72) Erfinder: **Toussaint, Herbert, Dr.**
**Knietschstrasse 11**
**D-6710 Frankenthal (DE)**
Erfinder: **Pander, Hans Joachim**
**Hochdorfer Strasse 19**
**D-6701 Roedersheim-Gronau (DE)**
Erfinder: **Gramlich, Walter, Dr.**
**Auf der Hoehe 11**
**D-6803 Edingen-Neckarhausen (DE)**
Erfinder: **Halbritter, Klaus, Dr.**
**Schwarzwaldstrasse 19**
**D-6800 Mannheim (DE)**
Erfinder: **Schulte, Wolfgang, Dr.**
**Barbarossastrasse 9**
**D-6733 Hassloch (DE)**

**EP 0 087 712 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft ein neues Verfahren zur Herstellung von aliphatischen Aldehyden, die in 3-Stellung eine Cyangruppe enthalten, sowie die Verwendung von 3-Cyan-2-ethylhexanal zur Herstellung von 2-Propylpentansäure.

Aus Houben-Weyl « Methoden der Organischen Chemie » Bd. 8, Seite 272 f ist bekannt, daß man 3-Cyanalkylketone durch Anlagerung von Blausäure an α,β-ungesättigte Ketone, z. B. Methylvinylketon, bei erhöhter Temperatur erhalten kann, während bei niedriger Temperatur nur die Cyanhydrine entstehen. Bei α,β-ungesättigten Aldehyden soll danach die analogue Umsetzung mit Blausäure ausschließlich zu Cyanhydrinen fühen, eine Anlagerung des Cyanwasserstoffs an die Doppelbindung erfolgt nicht. Gemäß der US-PS 2 565 537 wird diese Aussage dahingehend abgeschwächt, daß eine derartige Anlagerung doch erfolgen kann, wenn man Acrolein, Methacrolein oder Crotonaldehyd — also solche ungesättigte Aldehyde, die in α- und β-Stellung zusammen höchstens eine Alkylgruppe tragen — in einem niedrigen Alkohol gelöst bei 50 bis 100 °C innerhalb 1 bis 4 Stunden mit Blausäure umsetzt.

Aus der US-PS 3 444 161 ist schließlich bekannt, daß man α,β-ungesättigte Carbonylverbindungen dann zu 3-Cyancarbonylverbindungen umsetzen kann, wenn man die Blausäure in Form eines Cyanaluminiumkomplexes einsetzt. Diese Umsetzungen sind, wenn sie auch in kürzerer Zeit ablaufen, sehr umständlich auszuführen, da in einem zusätzlichen Arbeitsgang zunächst der Cyanaluminiumkomplex hergestellt werden muß.

Betreffend der Herstellung von 2-Propylpentansäure sind bisher verschiedene Methoden bekannt :

In den DE-OSen 27 21 264 und 27 21 265 wird ein Verfahren ausgehend von einem Ester der Cyanessigsäure beschrieben. Setzt man Cyanessigsäureester mit n-Propylbromid oder -iodid in Gegenwart von Natrium-n-propylat um, verseift den entstehenden Di-n-propyl-cyanessigsäureester mit Lauge und säuert dann an, so erhält man Di-n-propyl-cyanessigsäure. Durch Decarboxylierung und Verseifung des gebildeten 2-Propylpentannitril mit wäßriger Schwefelsäure erhält man die freie Säure.

Dieses Verfahren benötigt relativ unwirtschaftliche Ausgangsmaterialien.

Bei in der DE-OS 28 53 732 vorgeschlagenen ähnlichen Verfahren geht man anstelle von Cyanessigsäureester von Malonsäureester aus und setzt mit Propylhalogenid zum Dipropylmalonsäureester um, der alkalisch zum Salz der Dipropylmalonsäure verseift wird und nach Ansäuern durch Decarboxylierung 2-Propylpentansäure liefert. Ein Nachteil dieses Verfahrens ist neben dem teuren Ausgangsmaterial vor allem die Notwendigkeit, die als Zwischenprodukt auftretende Dipropylmalonsäure als Feststoff zu isolieren.

Nach der DE-OS 28 44 638 geht man von Buttersäure aus, die an einem Lanthanoxid/Aluminiumoxid-Katalysator unter Kohlendioxid-Abspaltung bei hoher Temperatur zum Heptan-4-on umgesetzt wird. Man reduziert zu Heptan-4-ol und dehydratisiert zu Hept-3-en. Hydroformylierung in Gegenwart von Ruthenium-(III)-chlorid und Trialkylphosphin liefert ein Isomerengemisch von 75 Teilen 2-Propylpentanal und 25 Teilen 2-Ethylhexanal, das mit Sauerstoff oxidiert wird. Das erhaltene Gemisch aus 2-Propylpentansäure und 2-Ethylhexansäure muß durch Destillation getrennt werden, wobei auf 2,3 Teile 2-Propylpentan säure 1 Teil 2-Ethylhexansäure anfällt.

Hauptnachteil dieses Verfahrens ist der Zwangsanfall eines weiteren Produkts, sog. Koppelproduktion, die durch die schlechte Selektivität der Hydroformylierung hervorgerufen wird sowie der große Destillationsaufwand zur Trennung der beiden Carbonsäuren ; hierzu ist nämlich eine Kolonne mit 90 theoretischen Böden nötig.

Das Ziel der Erfindung bestand nun darin, 3-Cyanaldehyde in einem möglichst einfachen Verfahren herzustellen, und weiterhin ein neues Verfahren zur Herstellung von 2-Propylpentansäure anzugeben, das von preisgünstigen, in technischen Mengen gut verfügbaren Ausgangsmaterialien ausgeht, das technisch problemlos durchführbare Reaktionsschritte enthält und das Zielprodukt in guter Ausbeute und Reinheit liefert.

Es wurde nun gefunden, daß man 3-Cyanaldehyde der allgemeinen Formel I

$$\begin{array}{ccccccc} & & CN & & R^2 & & \\ R^1 & - & C & - & C & - & CHO \\ & & R^3 & & H & & \end{array} \qquad (I)$$

in der $R^1$ und $R^2$ gleich oder verschieden sind und geradkettige oder verzweigte Alkylgruppen mit 1 bis 6 Kohlenstoffatomen und $R^3$ ein Wasserstoffatom oder eine Alkylgruppe mit 1 bis 3 Kohlenstoffatomen bedeuten, vorteilhaft erhält, wenn man einen α,β-ungesättigten Aldehyd der allgemeinen Formel II

$$\begin{array}{ccccccc} & & & & R^2 & & \\ R^1 & - & C & = & C & - & CHO \\ & & R^3 & & & & \end{array} \qquad (II)$$

in der $R^1$, $R^2$ und $R^3$ die oben genannte Bedeutung haben, ggf. in einem Lösungsmittel, in Gegenwart von 0,01 bis 1,0 Gew.-%, bezogen auf den Aldehyd, eines basischen Katalysators mit Blausäure vermischt und die Reaktionsmischung 1 bis 120 Minuten einer Temperatur von 100 bis 200 °C aussetzt.

Angesichts der Lehren in Houben-Weyl (s.o.) und der US-PS 2 565 537 mußte dieser Reaktionsweg überraschen, da man einerseits allenfalls eine Cyanhydrinbildung erwarten konnte und andererseits bei diesen hohen Temperaturen in alkalischem Medium eine Aldolkondensation als Konkurrenzreaktion befürchten mußte. Darauf wird in der US-PS 2 565 532 hingewiesen, wonach schon bei den darin empfohlenen niedrigeren Temperaturen der alkalische Katalysatoranteil möglichst gering gehalten werden soll, um die Polymerisation der Aldehyde zu vermeiden.

3-Cyan-2-ethylhexanal, das aus dem technischen Großprodukt 2-Ethylhex-2-enal mittels des erfindungsgemäßen Verfahrens leicht erhalten wird, kann vorteilhaft zur Herstellung von 2-Propylpentansäure verwendet werden. Man erhält diese Säure in einfacher Weise und in guten Ausbeuten durch Decarbonylierung von 3-Cyan-2-ethylhexanal zu einem Gemisch aus 4-Cyanhept-3-en und 4-Cyanheptan, gegebenenfalls mit anschließender Hydrierung dieses Gemischs zu reinem 4-Cyanheptan und schließlich Verseifung des 4-Cyanheptans zur 2-Propylpentansäure.

Aus Houben-Weyl Bd 7/1, Seite 494 f ist die Decarbonylierung von Aldehyden und aus Comprehensive Organic Chemistry Bd. 1 (1979), Seite 959 und Bd. 3 (1979), Seite 1 249 die Katalyse dieser Reaktion mittels Palladium bekannt.

Ausgangsverbindungen für die nach dem erfindungsgemäßen Verfahren herzustellenden 3-Cyanaldehyde sind α,β-ungesättigte Aldehyde der o. g. Formel II, in der $R^1$, $R^2$ und $R^3$ die definitionsgemäße Bedeutung besitzen. Vorzugsweise steht $R^1$ für eine Alkylgruppe mit 1 bis 4 Kohlenstoffatomen, $R^2$ für eine Alkylgruppe mit 1 bis 3 Kohlenstoffatomen und $R^3$ für ein Wasserstoffatom oder eine Methyl- oder Ethylgruppe. Die Alkylgruppen können geradkettig oder verzweigt sein.

Einzelne Vertreter für Verbindungen der Formel II sind beispielsweise 2-Methylbut-2-enal, 2-Methylpent-2-enal, 2-Methylhex-2-enal, 2-Methylhept-2-enal, 2-Ethylbut-2-enal, 2-Ethylpent-2-enal, 2-Ethylhex-2-enal, 2-Ethylhept-2-enal, 2-Ethyl-5-methylhex-2-enal, 2,3-Dimethylbut-2-enal, oder 2-Propylhept-2-enal.

Diese Aldehyde werden gegebenenfalls in einem Lösungsmittel gelöst, worauf der basische Katalysator in Mengen von 0,01 bis 1,0 Gew.%, vorzugsweise 0,05 bis 0,5 Gew.%, bezogen auf Aldehyd zugesetzt wird.

Lösungsmittel im Sinne der Erfindung sind stark polare Lösungsmittel, wie z. B. Isobutanol, Dimethylformamid, N,N-Dimethylacetamid, N-Methylpyrrolidon oder Dimethylsulfoxid. Zweckmäßigerweise soll die Menge des Lösungsmittels so bemessen werden, daß die Reaktionslösung 10 bis 90 Gew.%, vorzugsweise 25 bis 70 Gew.-% an Aldehyd enthält.

Als alkalischen Katalysator setzt man beispielsweise quaternäre Ammoniumbasen, Natrium- oder Kaliumhydroxid, Alkalialkoholate, wie Methanolat, Ethanolat oder t-Butanolat, oder bevorzugt Natrium- oder Kaliumethylenglycolat oder -propylenglycolat ein.

Eine Ausführungsform besteht darin, daß man der katalysatorhaltigen Aldehydlösung die zur Umsetzung erforderliche Menge an Blausäure zusetzt, wobei sich zunächst im Rahmen eines Gleichgewichts das Cyanhydrin bildet. Bevorzugt setzt man dabei Blausäure in geringem molaren Unterschuß, bezogen auf den Aldehyd, ein. Man kann aber auch schon vorher hergestelltes Aldehydcyanhydrin zusammen mit dem Katalysator in dem Lösungsmittel lösen, wobei die Menge an Cyanhydrin so zu bemessen ist, daß sie der obengenannten Aldehydmenge entspricht.

Die so erhaltenen Reaktionslösungen werden dan in geeigneten Reaktionsgefäßen 1 bis 120 Minuten, vorzugsweise 5 bis 40 Minuten lang einer Temperatur von 100 bis 200 °C, vorzugsweise 120 bis 160 °C ausgesetzt und anschließend wieder möglichst rasch abgekühlt.

Eine andere Ausführungsform besteht darin, eine Mischung aus Blausäure, Aldehyd und ggf. des Lösungsmittels nach Zugabe des Katalysators innerhalb der definierten Zeit auf der definierten Temperatur zu halten.

Um eine möglichst exakte Temperaturführung und definierte Verweilzeiten zu erreichen, wird das Verfahren vorzugsweise kontinuierlich durchgeführt. Hierbei geht man z. B. von einer katalysatorhaltigen Aldehydcyanhydrinlösung aus, die man kontinuierlich durch einen beheizten Rohrreaktor oder über eine Reaktorkaskade leitet. Die Verweilzeit soll definitionsgemäß von der obengenannten Dauer sein ; im allgemeinen kommt man mit Verweilzeiten von 5 bis 40 Minuten aus.

Wenn unter optimalen Bedingungen gearbeitet wird, erzielt man Ausbeuten von bis zu 90 % d. Th. Die aus dem Reaktor abgezogene Reaktionslösung wird, nach Zugabe einer Mineralsäure, wie Phosphorsäure zur Neutralisation des basischen Katalysators, in üblicher Weise aufgearbeitet.

Dies kann z. B. durch Vakuumdestillation erfolgen, wobei zunächst als niedersiedende Fraktion ein Gemisch aus Lösungsmittel und nicht umgesetztem, ungesättigtem Aldehyd abgezogen wird. Dieses Gemisch kann ggf. ohne weitere Reinigung wieder in die Reaktion zurückgeführt werden. Zur quantitativen Bestimmung des Aldehydanteils in der nieder siedenden Fraktion wird ein aliquoter Teil mit Wasser verdünnt, worauf sich der ungesättigte Aldehyd als organische Phase abscheidet. Als höher siedende Fraktion erhält man die gewünschten 3-Cyanaldehyde in hoher Reinheit.

Setzt man für die Umsetzung Lösungsmittel ein, die in jedem Verhältnis mit Wasser mischbar sind, so kann man die Reaktionslösung auch in Wasser geben, das zur Neutralisation des basischen Katalysators

einen sauren pH-Wert aufweisen soll. Dabei gelangt das Lösungsmittel in die wäßrige Phase, während die organische Phase den Cyanaldehyd und nicht umgesetzten ungesättigten Aldehyd enthält. Sie wird, ggf. nach Zusatz eines wasserunlöslichen Lösungsmittels, getrocknet und unter vermindertem Druck fraktioniert destilliert.

Das nach dieser Methode aus 2-Ethylhex-2-enal erhältliche 3-Cyan-2-ethylhexanal läßt sich dann z. B. in Gegenwart von Palladiumkatalysatoren in flüssiger Phase und in Abwesenheit von Lösungsmitteln bei 190 bis 250 °C, vorzugsweise 190 bis 220 °C, decarbonylieren, wobei Kohlenmonoxid und geringe Mengen an Wasserstoff entstehen. Als Träger für den Palladiumkatalysator eignen sich z. B. Tierkohle, Aktivkohle sowie Aluminiumoxid ; die besten Ergebnisse — eine Ausbeute von über 80 % — lieferte Aktivkohle mit einer Oberfläche von 800 bis 900 $m^2$/g. Der Palladiumgehalt kann z. B. 1 bis 25 Gew.%, vorteilhaft 1 bis 10 Gew.% sein ; der günstigste Anteil wurde zu etwa 5 Gew.% gefunden. Durch Abdestillieren eines Teils des Produktes während der Umsetzung läßt sich die Raum-Zeit-Ausbeute weiter steigern. Das restliche Produkt — i. a. mindestens ein Drittel — wird schließlich durch Destillation ebenfalls vom Rückstand getrennt ; der verbliebene Katalysator kann erneut eingesetzt werden.

Das erhaltene Produkt, ein Gemisch von 4-Cyanhept-3-en und 4-Cyanheptan (2-Propylpenten- bzw. -pentannitril) läßt sich vollständig, mit Vorteil ebenfalls in Gegenwart eines Palladiumkatalysators bei bis zu 100 °C, bevorzugt bei 30 bis 50 °C zu 4-Cyanheptan hydrieren. Träger des Palladiums kann ebenfalls Aktivkohle oder Tierkohle oder vorzugsweise Aluminiumoxid sein. Der Palladium-Gehalt des hier verwendeten Katalysators kann geringer als der des ersten Katalysators sein, z. B. zwischen 0,1 bis 10 Gew.% liegen. Bewährt haben sich auch Katalysatoren, die geringe Zusätze, z. B. Zink, Cadmium oder Praseodym, die ebenfalls im Bereich von 0,5 bis 6 Gew.% liegen können, enthalten. Solche Katalysatoren sind z. B. in der DE-OS 26 15 308 beschrieben. Man kann im Prinzip auch den für die Decarbonylierung benützten Katalysator verwenden, wenn die apparative Anordnung dies zuläßt ; die Hydrierung ist von der Art des verwendeten Katalysators relativ wenig abhängig.

Das rohe 4-Cyanheptan läßt sich dann ohne weitere Reinigung zur 2-Propylpentansäure verseifen. Die Verseifung läßt sich sowohl unter sauren (z. B. mit Schwefelsäure) wie unter basischen Bedingungen (NaOH oder KOH in Ethylenglykol) durchführen. die rohe Säure läßt sich leicht durch Destillation reinigen.

Die genannten 3-Cyanaldehyde stellen wertvolle Zwischenprodukte dar, die z. B. durch aminierende Hydrierung in 1,3-Diamine umgewandelt werden können, die wiederum als Reaktionskomponenten bei der Herstellung von z. B. Epoxidharzhärtern Verwendung finden.

2-Propylpentansäure und ihre Derivate besitzen als Psychopharmaka und Antiepileptika Bedeutung.

Die nun folgenden Beispiele erläutern die Erfindung. Angegebene Teile und Prozente sind, soweit sie nicht ausdrücklich anders bezeichnet werden, Gewichtsteile und -prozente. Raumteile verhalten sich zu Gewichtsteile wie Liter zu Kilogramm.

## Beispiel 1

In einem Rührautoklav wurden 195 Teile 2-Ethylhex-2-enal 97 %ig, 77 Teile N-Methylpyrrolidon und 33 Teile stabilisatorfreie Blausäure vorgelegt. Nach Verschließen des Autoklaven dosierte man im Verlauf von ca. 5 Minuten 18 Teile einer 5 %igen Lösung von Natriumhydroxid in Ethylenglykol zu. Dann wurde der Autoklav 40 Minuten lang auf 115 °C erhitzt. Nach Reaktionsende wurde das Umsetzungsprodukt über ein Steigrohr entnommen und auf eine Mischung aus 5 Teilen 85 %iger Phosphorsäure und 300 Teilen Eis gegeben. Die organische Phase wurde in 300 Teilen Diethylether aufgenommen und dreimal mit je 500 Teilen Wasser gewaschen. Es wurde über Magnesiumsulfat getrocknet und fraktioniert destilliert. Man erhielt 55 Teile 2-Ethylhex-2-enal und 117 Teile 3-Cyan-2-ethylhexanal in einem Siedebereich zwischen 35 bis 47 °C (0,05 mbar).

Ausbeute :

71,9 % d. Th. bezogen auf umgesetztes 2-Ethylhex-2-enal

62,6 % d. Th. bezogen auf eingesetzte Blausäure.

## Beispiel 2

In eine Lösung aus 1 820 Teilen 2-Ethylhex-2-enal (97 %ig), 1 820 Teilen N,N-Dimethylacetamid und 12 Teilen einer 5 %igen Lösung von Natriumhydroxid in Ethylenglykol wurden unter Kühlung bei 40 °C 303 Teile wasserfreie Blausäure eingegast. Das erhaltene Reaktionsgemisch wurde für die folgende kontinuierliche Reaktion eingesetzt.

In eine Kaskade aus drei heizbaren Rührkolben von je 50 Raumteilen Reaktionsvolumen wurden in den ersten Kolben stündlich 553 Teile der oben hergestellten Reaktionsmischung und 8 Teile einer 5 %igen Lösung von Natriumhydroxid in Ethylenglykol eindosiert. Die mittlere Verweilzeit lag bei ca. 5 Minuten pro Reaktor. Durch zwei Heizkreisläufe wurde die Innentemperatur des ersten Kolbens bei 130 °C, dies des zweiten und dritten Kolbens bei 140 °C gehalten. Das aus dem dritten Kolben ablaufende Reaktionsgemisch wurde auf 20 bis 30 °C abgekühlt und nach Zugabe von 5 Teilen 85 %iger Phosphorsäure pro Stunde gesammelt.

1 569 Teile des Reaktionsaustrages wurden in 1 000 Teilen Diethylether aufgenommen und dreimal

mit je 1 500 Teilen Wasser gewaschen. Die organische Phase wurde über Magnesiumsulfat getrocknet und nach Abtrennung des Lösungsmittels im Hochvakuum destilliert. Man erhielt 175 Teile nicht umgesetztes 2-Ethylhex-2-enal und 570 Teile 3-Cyan-2-ethylhexanal vom Siedepunkt 45 bis 47 °C (0,05 mbar), das entspricht einer Ausbeute von 92,3 % d. Th., bezogen auf umgesetztes 2-Ethylhex-2-enal bzw. 85,7 % d. Th., bezogen auf eingesetzte Blausäure. Die gaschromatographisch bestimmte Reinheit des Umsetzungsproduktes lag bei 99,5 %.

C : 70,4 % (ber. : 70,59 %) ; H : 9,9 % (ber. : 9,80 %) ;
N : 9,1 % (ber. : 9,15 %) ; O : 10,5 % (ber. : 10,46 %).

## Beispiel 3

Ein Compartmentreaktor, bestehend aus neun Compartments mit je Volumenteilen Inhalt, ist über zwei getrennte Ölkreisläufe beheizbar. Ölkreislauf 1 wurde auf 120 °C, Ölkreislauf 2 auf 150 °C eingestellt. Über zwei separate Pumpen wurden durch den Reaktor pro Stunde eine Mischung aus 235 Teilen 2-Ethylhex-2-enal, 235 Teilen N,N-Dimethylacetamid und 39 Teilen stabilisatorfreier Blausäure sowie 20 Teile einer 2,5 %igen Lösung von Natriumhydroxid in Ethylenglykol zudosiert. Das am Reaktorende ablaufende Reaktionsgemisch wurde auf 70 bis 80 °C abgekühlt und mit 13 Teilen 85 %iger Phosphorsäure pro Stunde versetzt. Die Lösung wurde auf einen Dünnfilmverdampfer geleitet, der bei einer Temperatur von 140 °C und einem Vakuum von 0,5 mbar betrieben wurde. Pro Stunde fielen 343 Teile Destillat an, die aus 51 Teilen 2-Ethylhex-2-enal, 153 Teilen 3-Cyan-2-ethylhexanal und 230 Teilen N,N-Dimethylacetamid bestanden.

Daraus berechnet sich eine Ausbeute von 69,2 % der Theorie, bezogen eingesetzte Blausäure bzw. 71,3 % der Theorie, bezogen auf ungesetztes 2-Ethylhex-2-enal.

## Beispiel 4

In die in Beispiel 2 beschriebene Kaskade aus drei Rührkolben wurde pro Stunde eine Mischung aus 201 Teilen 2-Methylpent-2-enal, 103 Teilen N-Methylpyrrolidon und 45 Teilen stabilisatorfreier Blausäure sowie 15 Teile einer 5 %igen Lösung von Natriumhydroxid in Ethylenglykol getrennt über zwei Pumpen zudosiert. Die mittlere Verweilzeit lag bei ca. 8 Minuten pro Kolben. Durch entsprechende Heizung wurde die Temperatur in den Reaktoren bei 120 °C gehalten. Das aus dem dritten Rührkolben ablaufende Reaktionsgemisch wurde auf 20 bis 30 °C abgekühlt und mit 4 Teilen 85 %iger Phosphorsäure pro Stunde versetzt.

761 Teile des Reaktionsaustrages wurden in 500 Teilen Diethylether gelöst. Die Lösung wurde dreimal mit je 500 Teilen Wasser gewaschen, die zurückbleibende organische Phase über Magnesiumsulfat getrocknet und fraktioniert destilliert. Nach Abtrennung des Diethylethers erhielt man 169 Teile 2-Methylpent-2-enal und 192 Teile 3-Cyan-2-methylpentanal vom Siedepunkt 95 bis 96 °C (11 mbar). Daraus erreichnet sich eine Ausbeute von 61 % d. Th., bezogen auf umgesetztes 2-Methylpent-2-enal bzw. 44,5 % d. Th., bezogen auf eingesetzte Blausäure.

C : 67,0 % ; ber. : 67,2 %
H : 8,9 % ; ber. : 8,8 %
N : 11,0 % ; ber. : 11,2 %
O : 13,1 % ; ber. : 12,8 %

Carbonylzahl 438 ; ber. : 449

## Beispiel 5

In eine Lösung aus 1 812 Teilen 2-Methylhex-2-enal, 825 Teilen N-Methylpyrrolidon und 83 Teilen einer 5 %igen Lösung von Natriumhydroxid in Ethylenglykol wurden bei 35 bis 40 °C 351 Teile wasserfreie Blausäure unter Kühlung eingegast. Das hierbei erhaltene Reaktionsgemisch wurde für die folgende kontinuierliche Reaktion eingesetzt.

In eine beheizbare Kaskade, die aus drei Rührkolben von je 50 Raumteilen Reaktionsvolumen besteht, wurden pro Stunde 378 Teile der oben hergestellten Reaktionsmischung zudosiert. Die mittlere Verweilzeit lag bei ca. 8 Minuten pro Reaktor. Durch getrennte Heizkreisläufe wurde die Reaktionstemperatur in den Kolben bei 130 °C gehalten. Das aus dem dritten Reaktionskolben ablaufende Reaktionsgemisch wurde nach dem Abkühlen mit 4 Teilen 95 %iger Phosphorsäure pro Stunde versetzt. 806 Teile der mit Phosphorsäure stabilisierten Reaktionslösung wurden in 400 Teilen Methyl-tert.-butylether aufgenommen und dreimal mit je 500 Teilen Wasser gewaschen. Die etherische Lösung wurde über Magnesiumsulfat getrocknet und nach Abtrennen des Methyl-tert.-butylethers im Valuum destilliert. Man erhielt 217 Teile 2-Methylhex-2-enal vom Siedepunkt 58 bis 59 °C (15 mbar) und 243 Teile 3-Cyan-2-methylhexanal vom Siedepunkt 44 bis 45 °C (0,1 mbar). Daraus errechnet sich eine Ausbeute von 77,1 % d. Th., bezogen auf umgesetztes 2-Methylhex-2-enal bzw. 51,8 % d. Th., bezogen auf eingesetzte Blausäure.

C : 68,8 % ; ber. : 69,06 %
H : 9,4 % ; ber. : 9,35 %
N : 10,4 % ; ber. : 10,07 %
O : 11,8 % ; ber. : 11,51 %

Carbonylzahl : 395 ; ber. : 403,7

Beispiel 6

In eine Mischung aus 1 801 Teilen 2-Ethyl-5-methylhex-2-enal, 4 042 Teilen N,N-Dimethylacetamid und 250 Teilen einer 4,5 %igen Lösung von Kaliumhydroxid in Ethylenglykol wurden unter Kühlung bei 40 °C 285 Teile Blausäure eingegast. Die erhaltene Lösung wurde für die im folgenden beschriebene kontinuierliche Reaktion eingesetzt.

In den im Beispiel 3 beschriebenen Compartmentreaktor wurden pro Stunde 1 058 Teile der oben hergestellten Reaktionsmischung zudosiert. Der Ölkreislauf 1 wurde auf 130 °C, der Kreislauf 2 auf 150 °C eingestellt. Die mittlere Verweilzeit lag bei 9,5 Minuten. Das am Reaktorende ablaufende Reaktionsgemisch wurde auf 20 bis 30 °C abgekühlt und mit 7 Teilen 85 %iger Phosphorsäure pro Stunde versetzt.

2 766 Teile des Austrages wurden filtriert und bei einer Heiztemperatur von 140 °C und einem Vakuum von 1 mbar in einem Dünnfilmverdampfer destilliert. Man erhielt 2 623 Teile Rohdestillat und 143 Teile Destillationsrückstand. Das Rohdestillat wurde im Hochvakuum redestilliert. Im Siedebereich von 21 bis 25 °C (0,5 mbar) fielen 1 502 Teile eines Gemisches aus N,N-Dimethylacetamid und 2-Ethyl-5-methylhex-2-enal an, aus dem durch Zusatz von 3 000 Teilen Wasser 160 Teile 2-Ethyl-5-methylhex-2-enal isoliert werden konnten. Als Umsetzungsprodukt erhielt man bei 55 bis 56 °C (0,5 mbar) 490 Teile 3-Cyan-2-ethyl-5-methylhexanal, das entspricht einer Ausbeute von 64,1 % d. Th., bezogen auf eingesetzte Blausäure bzw. 66,1 % bezogen auf umgesetztes 2-Ethyl-5-methylhex-2-enal.

C : 71,9 % ; ber. : 71,86 %
H : 10,1 % ; ber. : 10,18 %
N : 8,3 % ; ber. : 8,38 %
O : 9,7 % ; ber. : 9,58 %

Carbonylzahl : ber. : 336

Beispiel 7

In eine Mischung aus 1 775 Teilen 2-Methylhept-2-enal, 714 Teilen N-Methylpyrrolidon und 67 Teilen einer 5 %igen Lösung von NaOH in Ethylenglykol wurden im Verlauf von einer Stunde bei 40 °C 308 Teile Blausäure eingegast. Man ließ 15 Minuten bei 40 °C nachreagieren. Die erhaltene Reaktionslösung wurde abgekühlt und für die folgende kontinuierliche Reaktion eingesetzt.

In die in Beispiel 2 beschriebene Kaskade aus drei Rührkolben wurden pro Stunde 372 Teile der oben hergestellten Reaktionslösung zudosiert. Durch entsprechende Heizung wurde die Reaktionstemperatur in den drei Kolben bei 140 °C gehalten. Die mittlere Verweilzeit lag bei 8 Minuten pro Reaktor. 963 Teile des aus dem dritten Kolben ablaufenden, auf ca. 30 °C abgekühlten Umsetzungsproduktes wurden in 650 Teilen Diethylether aufgenommen und dreimal mit je 500 Teilen Wasser, dem 2 % Phosphorsäure zugesetzt wurde, gewaschen. Die organische Phase wurde über Magnesiumsulfat getrocknet. Nach destillativer Abtrennung des Diethylethers wurde das zurückbleibende Rohprodukt in einem Dünnfilmverdampfer destilliert. Man erhielt 550 Teile Destillat und 80 Teile Rückstand. Durch eine fraktionierte Redestillation wurden 285 Teile 2-Methylhept-2-enal (Siedepunkt : 60 bis 62 °C (13 mbar)) und 297 Teile 3-Cyan-2-methylheptanal (Siedepunkt : 111 bis 112 °C (13 mbar)) erhalten, das entspricht einer Ausbeute von 78,3 % d. Th., bezogen auf umgesetztes 2-Methylhept-2-enal bzw. 50,6 % d. Th., bezogen auf eingesetzte Blausäure.

C : 70,7 % ; ber. : 70,59 %
H : 10,0 % ; ber. : 9,80 %
N : 9,3 % ; ber. : 9,15 %
O : 10,3 % ; ber. : 10,46 %

Carbonylzahl : 366, ber. : 366,7

Beispiel 8

In eine Mischung aus 1 454 Teilen 2-Propylhept-2-enal, 1 482 Teilen N,N-Dimethylacetamid und 6 Teilen einer 4,8 %igen Lösung von Natriumhydroxid in Ethylenglykol wurden im Verlauf von etwa einer Stunde bei 40 °C 205 Teile Blausäure eingegast. Das Reaktionsgemisch wurde auf 20 °C abgekühlt und

nach Zugabe von 81 Teilen einer 4,8 %igen Lösung von Natriumhydroxid in Ethylenglykol für die folgende kontinuierliche Reaktion eingesetzt.

In den in Beispiel 3 beschriebenen Compartmentreaktor wurden über eine Pumpe pro Stunde 553 Teile der katalysatorhaltigen Reaktionslösung zudosiert. Der Ölkreislauf 1 wurauf 140 °C, der Kreislauf 2 auf 160 °C eingestellt. Die mittlere Verweilzeit lag bei 18 Minuten. Das am Reaktorende mit einer Temperatur von 155 °C austretende Reaktionsgemisch wurde auf 30 °C abgekühlt und mit 5 Teilen 85 %iger Phosphorsäure pro Stunde versetzt. 1 581 Teile des Reaktionsgemisches wurden filtriert und bei 0,5 mbar und 140 °C über einen Dünnfilmverdampfer destilliert. Man erhielt 1 463 Teile eines schwach gelb gefärbten Destillats, das über eine kurze Kolonne redestilliert wurde. Nach Abtrennung von 972 Teilen eines Gemisches aus N,N-Dimethylacetamid und 2-Propylhept-2-enal (Siedepunkt 22 bis 33 °C (0,5 mbar)) erhält man 451 Teile 3-Cyan-2-propylheptanal vom Siedepunkt 78 bis 79 °C (0,5 mbar). Aus der ersten Fraktion konnten durch Zugabe von 1 200 Teilen Wasser 254 Teile nicht umgesetztes 2-Propylhept-2-enal zurückgewonnen werden. Daraus errechnet sich eine Ausbeute von 67,6 % d. Th., bezogen auf eingesetzte Blausäure bzw. 84,9 % d. Th., bezogen auf umgesetztes 2-Propylhept-2-enal.

C : 72,7 % ; ber. : 72,93 %
H : 10,6 % ; ber. : 10,50 %
N : 7,6 % ; ber. : 7,73 %
O : 8,9 % ; ber. : 8,84 %

Carbonylzahl : 314 ; ber. : 310

## Beispiel 9

Zu einer Mischung aus 712 Teilen 2,3-Dimethylbut-2-enal, 1 424 Teilen N,N-Dimethylacetamid und 16 Teilen einer 1 %igen Lösung von Natriumhydroxid in Ethylenglykol wurden unter Kühlung bei 40 °C portionsweise 160 Teile frisch destillierte Blausäure zugegeben. Das Reaktionsgemisch wurde abgekühlt, mit 48 Teilen einer 4,8 %igen Lösung Natriumhydroxid in Glykol versetzt und für die im folgenden beschriebene kontinuierliche Reaktion eingesetzt.

In den im Beispiel 3 beschriebenen Compartmentreaktor wurden über eine Dosierpumpe pro Stunde 564 Teile der oben hergestellten Reaktionsmischung zudosiert. Ölkreislauf 1 des Reaktors wurde auf 120 °C, Kreislauf 2 auf 150 °C eingestellt. Die mittlere Verweilzeit lag bei 18 Minuten. Das am Reaktorende ablaufende Reaktionsprodukt wurde pro Stunde mit 5 Teilen 85 %iger Phosphorsäure versetzt.

854 Teile des Reaktionsgemisches wurden auf 1 000 Teile Wasser gegossen. Das aufschwimmende Öl wurde in 300 Teilen Diethylether aufgenommen ; die wäßrige Phase noch zweimal mit je 150 Teilen Diethylether extrahiert. Die vereinigten Extrakte wurden einmal mit 200 Teilen Wasser gewaschen, über Magnesiumsulfat getrocknet und destilliert. Nach Abtrennung des Lösungsmittels und nicht umgesetztem 2,3-Dimethylbut-2-enal erhält man 158 Teile 3-Cyan-2,3-dimethylbutanal vom Siedepunkt 79 bis 80 °C (13 mbar), das entspricht einer Ausbeute von 59,4 % d. Th., bezogen auf eingesetzte Blausäure.

C : 67,1 % ; ber. : 67,20 %
H : 8,6 % ; ber. : 8,80 %
N : 11,3 % ; ber. : 11,20 %
O : 12,9 % ; ber. : 12,80 %

## Beispiel 10

Es wurden 500 Teile des nach der Vorschrift des Beispiel 1 gewonnenen 3-Cyan-2-ethylhexanals und 50 Teile eines Katalysators, der 5 % Palladium auf Aktivkohle enthält, vorgelegt und innerhalb von 45 Minuten auf 200 °C (gemessen in der Flüssigkeit) erhitzt.

Die nun beginnende Decarbonylierung konnte, wenn man wollte, durch Erhöhung der Temperatur auf 210 bis 220 °C beschleunigt werden. Das entstehende Gemisch aus 4-Cyanhept-3-en/4-Cyanheptan konnte während der Reaktion bereits abdestilliert werden (Kp 183 °C) ; es hat sich jedoch gezeigt, daß nicht mehr als etwa 2 Drittel der Gesamtmenge entfernt werden soll, da sonst der Katalysator für weitere Umsetzungen unbrauchbar werden kann. Nach 3 bis 5 Stunden war die Reaktion beendet. Nach Abtrennen vom Katalysator wurde destilliert. Man erhielt 301 Teile einer Mischung von 4-Cyanhept-3-en und 4-Cyanheptan, was einer Ausbeute von 74 % d. Th. entspricht. Der Anteil an ungesättigtem Nitril lag bei 20 %.

## Beispiel 11

In einem Autoklaven wurden 300 Teile des Gemisches der Nitrile (Beispiel 10) sowie 15 Teile eines Aluminiumoxid-Trägerkatalysators, der 0,5 % Palladium enthielt, 3 Stunden bei 35 °C und einem

Wasserstoffdruck von 20 bar hydriert. Das erhaltene 4-Cyanhpetan (2-Propylpentannitril) kann ohne weitere Reinigung für die nächste Stufe eingesetzt werden (Ausbeute über 99 %).

Beispiel 12

Man erhitzte ein Gemisch aus 250 Teilen 4-Cyanheptan, 100 Teilen Ethylenglykol und 86 Teilen NaOH auf 140 °C, bis die Ammoniakentwicklung beendet war (ca. 6 Stunden). Danach wurde mit 750 Teilen 12 %iger Salzsäure angesäuert, zur besseren Phasentrennung noch 50 Teilen Diethylether zugesetzt und nach Phasentrennung die organische Phase destilliert. Man erhielt 249 g (86 % d. Th) an reiner 2-Propylpentansäure.

**Patentansprüche**

1. Verfahren zur Herstellung von Verbindungen der allgemeinen Formel I

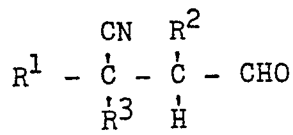

(I)

in der $R^1$ und $R^2$ gleich oder verschieden sind und geradkettige oder verzweigte Alkylgruppen mit 1 bis 6 Kohlenstoffatomen und $R^3$ ein Wasserstoffatom oder eine Alkylgruppe mit 1 bis 3 Kohlenstoffatomen bedeuten, dadurch gekennzeichnet, daß man einen ungesättigten Aldehyd der allgemeinen Formel II

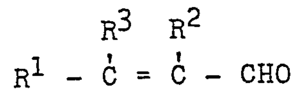

(II)

in der $R^1$, $R^2$ und $R^3$ die oben genannte Bedeutung besitzen, ggf. in einem Lösungsmittel, in Gegenwart von 0,01 bis 1,0 Gew.-%, bezogen auf den Aldehyd, eines basischen Katalysators mit Blausäure vermischt und die Reaktionsmischung 1 bis 120 Minuten einer Temperatur von 100 bis 200 °C aussetzt.

2. Verfahren gemäß Anspruch 1, dadurch gekennzeichnet, daß man den Aldehyd mit Blausäure zunächst zum Cyanhydrin umsetzt und dieses in Gegenwart des Katalysators in dem Lösungsmittel der Hitzebehandlung unterwirft.

3. Verfahren gemäß Anspruch 2, dadurch gekennzeichnet, daß man die Reaktion kontinuierlich führt.

4. Verwendung von 3-Cyan-2-ethylhexanal, erhalten gemäß Anspruch 1 aus 2-Ethylhex-2-enal, zur Herstellung von 2-Propylpentansäure durch Decarbonylierung von 3-Cyan-2-ethylhexanal zu einem Gemisch aus 4-Cyanhept-3-en und 4-Cyanheptan, gegebenenfalls mit anschließender Hydrierung dieses Gemischs zu reinem 4-Cyanheptan und schließlich Verseifung des 4-Cyanheptans zur 2-Propylpentansäure.

**Claims**

1. A process for the preparation of a compound of the general formula I

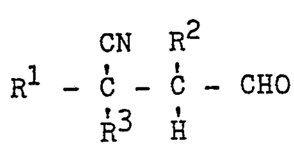

(I)

where $R^1$ and $R^2$ are identical or different and are each straight-chain or branched alkyl of 1 to 6 carbon atoms and $R^3$ is hydrogen or alkyl of 1 to 3 carbon atoms, wherein an unsaturated aldehyde of the general formula II

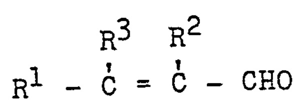

(II)

where $R^1$, $R^2$ and $R^3$ have the above meanings, if desired in a solvent, is mixed with hydrocyanic acid in the presence of from 0.01 to 1.0 % by weight, based on the aldehyde, of a basic catalyst, and the reaction mixture is heated at from 100 to 200 °C for from 1 to 120 minutes.

2. A process as claimed in claim 1, wherein the aldehyde is reacted with hydrocyanic acid to give a cyanohydrin as the initial product, and this is heated in the presence of the catalyst, in the solvent.

3. A process as claimed in claim 2, wherein the reaction is carried out continuously.

4. The use of 3-cyano-2-ethylhexanal, obtained as claimed in claim 1 from 2-ethylhex-2-enal, for the preparation of 2-propylpentanoic acid by decarbonylating 3-cyano-2-ethylhexanal to a mixture of 4-cyanohept-3-ene and 4-cyanoheptane, and then optionally hydrogenating this mixture to pure 4-cyanoheptanbe, and finally hydrolyzing the 4-cyanoheptane to 2-propylpentanoic acid.

## Revendications

1. Procédé de préparation de composés de la formule générale I

$$R^1 - \overset{\displaystyle CN}{\underset{\displaystyle R^3}{C}} - \overset{\displaystyle R^2}{\underset{\displaystyle H}{C}} - CHO \qquad (I)$$

dans laquelle $R^1$ et $R^2$, qui peuvent être identiques ou différents, désignent des radicaux alkyle en $C_1$ à $C_6$ à chaîne droite ou ramifiée et $R^3$ désigne un atome d'hydrogène ou un radical alkyle en $C_1$ à $C_3$, caractérisé en ce que l'on mélange un aldéhyde non saturé de la formule générale II

$$R^1 - \overset{\displaystyle R^3}{C} = \overset{\displaystyle R^2}{C} - CHO \qquad (II)$$

dans laquelle $R^1$, $R^2$ et $R^3$ possèdent la signification définie, éventuellement dans un solvant et en présence de 0,01 à 1,0 % du poids de l'aldéhyde d'un catalyseur basique, avec de l'acide cyanhydrique et on porte ce mélange pendant 1 à 120 minutes à une température comprise entre 100 et 200 °C.

2. Procédé suivant la revendication 1, caractérisé en ce que l'on transforme d'abord l'aldéhyde par réaction avec l'acide cyanhydrique en cyanhydrine, puis on soumet celle-ci dans le solvant au traitement thermique en présence du catalyseur.

3. Procédé suivant la revendication 2, caractérisé en ce que la réaction est effectuée en continu.

4. Utilisation du cyano-3 éthyl-2 hexanal, obtenu par le procédé de la revendication 1 à partir de l'éthyl-2 hexénal-2, pour la préparation de l'acide propyl-2 pentanoïque par décarboxylation du cyano-3 éthyl-2 hexanal en un mélange de cyano-4 heptène-3 et de cyano-4 heptane, suivie éventuellement d'une hydrogénation de ce mélange en cyano-4 heptane pur, puis de la saponification du cyano-4 heptane en acide propyl-2 pentanoïque.